# EUROPEAN PATENT APPLICATION

(11) **EP 2 400 027 A1**
(43) Date of publication of application: **28.12.2011**
(21) Application number: 11176367.8
(22) Date of filing: 31.10.2006
(51) Int. Cl.: C12P 7/18, C12N 1/15

(54) **Process for the biological production of 1,3-propanediol from glycerol with high yield**

(62) Divisional of application: 06819204.6
(71) Applicant: Metabolic Explorer, 63360 Saint Beauzire (FR)
(72) Inventor: Soucaille, Philippe, 31450 DEYME (FR)
(74) Representative: Tetaz, Franck Claude Edouard

(57) **Abstract**

The present invention provides a method for the anaerobic production of 1, 3 propanediol, by culturing a *Clostridium* strain in an appropriate culture medium comprising glycerol as a source of carbon, wherein said *Clostridium* strain does not produce substantially other products of the glycerol metabolism selected among the group consisting of: butyrate, lactate, butanol and ethanol, and recovering of 1,3-propanediol.

## Description

### FIELD OF INVENTION

The invention comprises a process for the bioconversion of glycerol to 1,3-propanediol at high yield by a metabolically engineered *Clostridium.*

### BACKGROUND OF THE INVENTION

1,3-propanediol is a monomer use in the production of polyester fibers and with potential use in the manufacture of polyurethanes and cyclic compounds.

1,3-propanediol can be produced by different chemical routes from i) acrolein water and hydrogen ii) ethylene oxide carbon monoxide and water in the presence of phosphine and from glycerol and hydrogen in the presence of carbon monoxide. All these methods have in common to be expensive and to generate waste streams containing polluting substances.

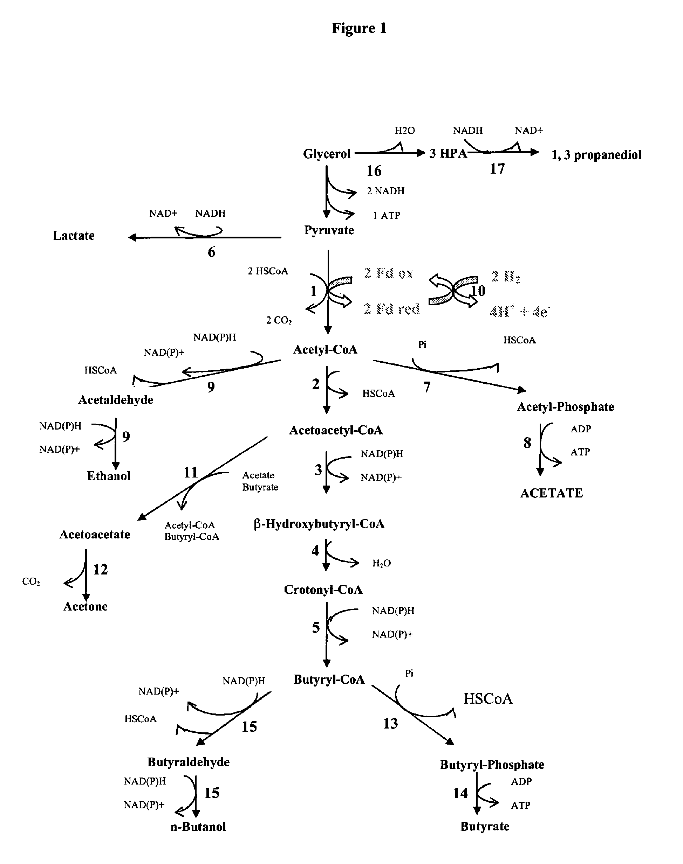

1,3-propanediol can be produced as an acetate/butyrate/lactate/1,3-propanediol mixture by the fermentation of glycerol by different *Clostridia.* The general metabolism of glycerol into *Clostridia* is presented in figure 1.

In one way, glycerol is converted to 1,3-propanediol in a two step enzymatic reaction sequence. In a first step a glycerol dehydratase catalyze the conversion of glycerol to 3-hydroxypropionaldehyde (3-HPA) and water. In the second step 3-HPA is reduced to 1,3-propanediol by a NADH dependent 1,3-propanediol dehydrogenase. Most of the 1,3-propanediol producing clostridia use a B12 dependent glycerol dehydratase encoded by the *dhaB1B2B3* structural genes while *Clostridum butyricum* uses a B12 independent enzyme encoded by the dhaB1 structural gene. For the B12 dependent glycerol dehydratases, orfX and orfZ encode the glycerol dehydratase reactivation factor while for the only known B12 independent enzyme, dhaB2 encodes an S-Adenosyl-Méthionine (SAM) dependent activation factor. Near the genes encoding the structural and activation factors a gene encoding a 1,3-propanediol dehydrogenase (dhaT) is also present. Production of 1,3-propanediol from glycerol consumes NADH.

In another way, when glycerol is not transformed into 1,3-propanediol, it is oxidized to dihydrohycetone-phosphate (DHAP) with the concomitant production of NADH by either a glycerol kinase and a glycerol-3-Phosphate dehydrogenase encoded respectively by *glpk* and *glpA* or by a glycerol dehydrogenase followed by a DHA kinase encoded respectively by *dhaD* and *dhaK1K2.* DHAP will then enter the glycolitic pathway with the production of pyruvate and acetyl-CoA as key intermediates. Pyruvate and acetyl-CoA can be reduced to respectively lactate and ethanol by a lactate dehydrogenase encoded by the *ldh* gene and a bi-functional aldehyde-alcohol dehydrogenases encoded by *adhE.* Acetyl-CoA can also be converted to butyryl-CoA, an intermediate product that can be:
i) converted to butyric acid by a phospho-transbutyrylase and a butyrate kinase encoded respectively by the *ptb* and *buk* genes or
ii) reduced to butanol by a bi-functional aldehyde-alcohol dehydrogenase encoded by *adhE.*

In solventogenic clostridia, acetone is produced from aceto-acetyl-CoA (an intermediate in the production of butyryl-CoA) by a CoA-transferase and an acetoacetate decarboxylase encoded respectively by the *ctfAB* and *adc* genes. Hydrogen is produced by an iron only hydrogenase encoded by the *hydA* gene.

Both natural and recombinant clostridia produce 1,3-propanediol at a maximal yield of 0.55 g/g of glycerol due to the co-production of reduced compounds like butyric acid (butyrate), lactic acid (lactate), ethanol or butanol. To increase the yield of 1,3-propanediol production it is necessary to avoid the production of all the reduced co-products and associate the production of 1,3-propanediol to an oxidized co-product.

*Clostridium acetobutylicum* strains unable to produce butyrate have already been described in the article (Green et al., 1996). The butyrate formation was dramatically reduced because of the inactivation of the *buk* gene obtained by single crossing-over with a non-replicable plasmid. This mutant strain was tested for the production of 1,3-propanediol as shown in (Gonzalez-Pajuelo, 2005, Metabolic Engineering). This recombinant strain effectively produces 1,3-propanediol as the main fermentation product, but produces also butanol, which decreases the 1,3-propanediol yield.

The 1,3-propanediol fermentation of glycerol by *Clostridia* can run in batch, fedbatch or continuous cultures.

The problem to be solved by the present invention is the biological production of 1,3 propanediol from glycerol at high yield, with no concomitant production of reduced compounds such as butyrate, lactate, or alcohols. This production is performed by anaerobic fermentation with *Clostridia.*

### SUMMARY OF THE INVENTION

Applicants have solved the stated problem and the present invention provides a method for the anaerobic production of 1, 3 propanediol, by culturing a *Clostridium* strain in an appropriate culture medium comprising glycerol as a source of carbon, wherein said *Clostridium* strain does not produce substantially other products of the glycerol metabolism selected among the group consisting of: butyrate, lactate, butanol and ethanol, and recovering 1,3-propanediol.

The 1,3-propanediol may be produced concomitantly with a single oxidized product of the glycerol metabolism.

In a particular aspect of the invention, the *Clostridium* strain is modified to limit production of metabolites from glycerol, which biosynthesis pathway is NADH or NADPH consuming, except for 1,3-propanediol.

In one aspect of this invention, a *Clostridium* naturally producing 1,3-propanediol is genetically modified to produce 1,3-propanediol at higher yield by deleting:
i) the gene coding for the butyrate kinase (*buk*) or the phospho-transbutyrylase (*ptb*) to avoid butyrate production
ii) optionally, all the genes coding for the lactate dehydrogenases (*ldh*) to avoid lactate production
iii) optionally, the genes coding for the bi-functional aldehyde-alcohol dehydrogenases (*adhE*) to avoid alcohols formation.

In another aspect of this invention, a *Clostridium* naturally producing butyrate but unable to produce 1,3-propanediol is genetically modified to produce 1,3-propanediol at high yield. This result is achieved by replacing the *ptb* or the *buk* genes coding for enzymes involved in the butyrate pathway with the operon of *C*. *butyricum* coding for enzymes involved in the B12 independent 1,3-propanediol pathway, and by deleting :
i) optionally, all the genes coding for the lactate dehydrogenases (*ldh*) to avoid lactate production
ii) optionally, the genes coding for the bi-functionnal aldehyde-alcohol dehydrogenases (*adhE*) to avoid alcohols formation.

In a further aspect of this invention, a *Clostridium* naturally producing ethanol but unable to produce 1,3-propanediol is genetically modified to produce 1,3-propanediol. This result is achieved by replacing one of the *adhE* genes coding for enzymes involved in the ethanol pathway, with the operon of *C*. *butyricum* coding for enzymes involved in the B12 independent 1,3-propanediol pathway, and by deleting:
i) optionally, all the genes coding for the lactate dehydrogenase (*ldh*) to avoid lactate production
ii) optionally, all the remaining genes coding for the bi-functionnal aldehyde-alcohol dehydrogenases (*adhE*) to avoid alcohol formation.

In another aspect of this invention, the flux of hydrogen production is decreased and then the flux of reducing equivalent is redirected toward 1,3-propanediol production by attenuating the gene encoding the hydrogenase (*hydA*).

In another aspect of the invention, the flux of 1,3-propanediol production is increased by introducing extra copies of the 1,3-propanediol operon from *C*. *butyricum,* (coding for enzymes involved in the B12 independent 1,3-propanediol pathway).

It is also an object of the present invention to provide a recombinant *Clostridum* strain, useful for the process of production of 1,3-propanediol at high yield.

### BRIEF DESCRIPTION OF THE DRAWINGS

The accompanying drawing which is incorporated in and constitutes a part of this specification exemplifies the invention and together with the description, serve to explain the principles of this invention.

**Figure 1** depicts the central metabolism of different *Clostridia.*
1 : Pyruvate-ferredoxin oxydoreductase ; 2 : Thiolase ; 3 : β-Hydroxybutyryl-CoA dehydrogenase ; 4 : Crotonase ; 5 : Butyryl-CoA dehydrogenase ; 6 : Lactate dehydrogenase ; 7 : Phospho-transacetylase ; 8 : Acetate kinase ; 9 : Acetaldehyde Ethanol deshydrogenase ; 10 : hydrogenase.; 11: CoA transférase (Acetoacetyl-CoA :acetate/butyrate : CoA transferase) ; 12 : Acetoacetate decarboxylase ; 13 : Phospho-transbutyrylase ; 14 : Butyrate kinase ; 15 : Butyraldehyde-Butanol dehydrogenase ; 16 : Glycerol dehydratase; 17: 1, 3 propanediol dehydrogenase.

### DETAILED DESCRIPTION OF THE INVENTION

As used herein the following terms may be used for interpretation of the claims and specification.

The terms *"Clostridium"* and *"Clostridia"* refer to all kind of bacteria belonging to this family.

An appropriate culture medium refers to a culture medium optimized for the growth and the diol production of the specifically used *Clostridium* strain.

The term "carbon substrate" or "source of carbon" means any carbon source capable of being metabolized by a microorganism wherein the substrate contains at least one carbon atom. In the present invention glycerol is the single source of carbon.

The phrase "microorganism is modified" means that the strain has been transformed in the aim to change its genetic characteristics. Endogenous genes can either be attenuated, deleted, or over-expressed. Exogenous genes can be introduced, carried by a plasmid, or integrated into the genome of the strain, to be expressed into the cell.

The term "attenuation" refers to a decreased expression of a gene or a decreased activity of the protein, product of the gene. The man skilled in the art knows numerous means to obtain this result, and for example:
- Introduction of a mutation into the gene, decreasing the expression level of this gene, or the level of activity of the encoded protein.
- Replacement of the natural promoter of the gene by a low strength promoter, resulting in a lower expression.
- Use of elements destabilizing the corresponding messenger RNA or the protein.
- Deletion of the gene if no expression is needed.

The term "deleted gene" means that a substantial part of the coding sequences of said gene was removed. Preferably, at least 50% of the coding sequence was removed, and more preferably at least 80%.

The term "plasmid" or "vector" as used herein refers to an extra chromosomal element often carrying genes which are not part of the central metabolism of the cell, and usually in the form of circular double-stranded DNA molecules.

In the description of the present invention, enzymes are identified by their specific activities. This definition thus includes all polypeptides that have the defined specific activity also present in other organisms, more particularly in other microorganisms. Often enzymes with similar activities can be identified by their grouping to certain families defined as PFAM or COG.

PFAM (protein families database of alignments and hidden Markov models; http://www.sanger.ac.uk/Software/Pfam/) represents a large collection of protein sequence alignments. Each PFAM makes it possible to visualize multiple alignments, see protein domains, evaluate distribution among organisms, gain access to other databases, and visualize known protein structures.

COGs (clusters of orthologous groups of proteins; http://www.ncbi.nlm.nih.gov/COG/) are obtained by comparing protein sequences from 43 fully sequenced genomes representing 30 major phylogenic lines. Each COG is defined from at least three lines, which permits the identification of former conserved domains.

The means of identifying homologous sequences and their percentage homologies are well known to those skilled in the art, and include in particular the BLAST programs, which can be used from the website http://www.ncbi.nlm.nih.gov/BLAST/ with the default parameters indicated on that website. The sequences obtained can then be exploited (e.g., aligned) using, for example, the programs CLUSTALW (http://www.ebi.ac.uk/clustalw/) or MULTALIN (http://prodes.toulouse.inra.fr/multalin/cgi- bin/multalin.pl), with the default parameters indicated on those websites.

Using the references given on GenBank for known genes, those skilled in the art are able to determine the equivalent genes in other organisms, bacterial strains, yeasts, fungi, mammals, plants, etc. This routine work is advantageously done using consensus sequences that can be determined by carrying out sequence alignments with genes derived from other microorganisms, and designing degenerate probes to clone the corresponding gene in another organism. These routine methods of molecular biology are well known to those skilled in the art, and are described, for example, in Sambrook et al. (1989 Molecular Cloning: a Laboratory Manual. 2nd ed. Cold Spring Harbor Lab., Cold Spring Harbor, New York.).

The present invention provides a method for the anaerobic production of 1,3-propanediol, by culturing a *Clostridium* strain in an appropriate culture medium comprising glycerol as a source of carbon, wherein said *Clostridium* strain does not produce substantially other products of the glycerol metabolism selected among the group consisting of: butyrate, lactate, butanol and ethanol, and recovering 1,3-propanediol.

"Substantially" means that at most traces of products or reductions of glycerol are found in the culture medium. Traces means preferably amounts that shall not interfere with the recovery process of 1,3-propanediol, more preferably less than 10 mM.

The phrase "glycerol metabolism" refers to all biochemical modifications of glycerol happening in the bacteria. This includes the biosynthesis of organic molecules (anabolism) and their breakdown (catabolism). Some metabolic reactions are consuming and some others are producing NADH/NADPH. Glycerol metabolism in *Clostridium* strains is illustrated in figure 1. Intermediates as well as final products from metabolic reactions are called metabolites.

The method of the invention is characterized by the fact that the glycerol metabolism is directed to 13,-propanediol production, and that no other reduced products from this metabolism pathway, such as butyrate, lactate, butanol, ethanol, are produced concomitantly with 1,3-propanediol by the *Clostridium.* Indeed, production of these reduced products is consuming NADH/NADPH stock of the cell. Limiting this consumption will allow the reducing power to be redirected toward 1,3-propanediol production.

In a specific embodiment of the invention, the 1,3-propanediol is produced concomitantly with a single oxidized product of glycerol metabolism, such as acetate, acetone or carbon dioxide. The term "oxidized product" refers to products produced without consumption of the NADH/NADPH stock of the cell

Advantageously, the *Clostridium* strain used in the process produces only 1,3-propanediol and acetate.

According to the invention, the *Clostridium* strain can be modified to limit production of metabolites from glycerol, which biosynthesis pathway is NADH or NADPH consuming, except for 1,3-propanediol.

Advantageously, this modification consists of the deletion of at least one gene coding for an enzyme involved in production of said metabolites.

In particular, this enzyme is involved in the production of a metabolite selected among the group consisting of : butyrate, lactate, butanol and ethanol.

In a specific embodiment of the invention , the *Clostridium* is naturally producing 1,3-propanediol, since it comprises functional endogenous genes encoding for enzymes involved in biosynthesis of 1,3-propanediol. These genes are in particular: glycerol dehydratase and 1,3-propanediol dehydrogenase.

This strain can be genetically modified to produce 1,3-propanediol as major product by deleting at least one gene encoding for phospho-transbutyrylase (*ptb*) or butyrate kinase (*buk*) to block the conversion of butyryl-CoA to butyrate.

In another specific embodiment, said *Clostridium* said was also deleted of all the genes encoding for lactate dehydrogenase *(ldh)* to block the production of lactate.

In another specific embodiment, said *Clostridium* said was also deleted of all the genes encoding for bifunctionnal aldehyde-alcohol dehydrogenases *(adhE)* to block the production of alcohols.

Deletion of genes in *Clostridia* can be done using the method recently described in patent application PCT/EP2006/066997 allowing the i) replacement of the gene to delete with an erythromycin resistance gene and ii) removal of the erythromycin resistance gene by expressing the FLP recombinase.

Advantageously, the *Clostridium* strain is selected among the group consisting of *C. butyricum* and *C*. *pasteurianum.*

In a specific embodiment of the invention, the *Clostridium* strain has to be modified to be able to produce 1,3-propanediol. The modification consists of the introduction of at least one heterologous gene coding for an enzyme involved in the B-12 independent 1,3-propanediol pathway. These genes may be but are not limited to dhaB1, dhaB2, dhaT.

Advantageously, the strain is modified by introducing the operon of *Clostridium butyricum* coding for the enzymes involved in the B12-independent 1,3-propanediol pathway. Insertion of the operon in the chromosome can be done using the method recently described in patent application PCT/EP2006/066997.

In a specific embodiment of the invention, the used *Clostridum* strain naturally produces butyrate but is unable to produce 1,3-propanediol prior modification; this specific *Clostridium* is genetically modified to produce 1,3-propanediol by replacing at least one gene encoding for an enzyme involved in butyrate formation, in particular the phospho-transbutyrylase (*ptb*) or the butyrate kinase (*buk*), with one heterologous gene coding for an enzyme involved in the B-12 independent 1,3-propanediol pathway in the aim to :
- block the conversion of butyryl-CoA to butyrate and
- allow 1,3-propanediol production from glycerol in this strain.

Insertion of the operon in the chromosome and deletion of the genes can be done using the method recently described in patent application PCT/EP2006/066997.

Preferentially, in this *Clostridium* strain, all the genes encoding for lactate dehydrogenase *(ldh)* are deleted to block the production of lactate.

Preferentially, in this *Clostridium* strain, all the genes encoding for bi-functional aldehyde-alcohol dehydrogenases (*adhE*) are deleted, to inhibit the production of alcohols.

Avantageously, this *Clostridium* strain is selected among the group consisting of *C*. *acetobutylicum, C. beijerinckii, C. saccharoperbutylacetonicum C. saccharobutylicum, C. butyricum* or *C*. *cellulolyticum.*

In a specific embodiment of the invention, the *Clostridum* naturally produces ethanol but is unable to produce 1,3-propanediol prior modification; this strain is genetically modified to produce 1,3-propanediol by replacing at least one gene encoding for bi-functional aldehyde-alcohol dehydrogenases (*adhE*) with at least one of the heterologous gene coding for an enzyme involved in the B12 independent 1,3-propanediol pathway. Preferentially this heterologous gene is the operon of *C*. *butyricum* encoding for enzymes involved in the B12 independent 1,3-propanediol pathway.

This replacement leads to :
- a decrease of the conversion of acetyl-CoA to ethanol, and
- 1,3-propanediol production from glycerol.

Preferably, in this *Clostridium* strain, all the genes encoding for lactate dehydrogenase (*ldh*) are deleted to inhibit the production of lactate.

Preferably, in this *Clostridium* strain, all the remaining genes encoding for bi-functional aldehyde-alcohol dehydrogenases (*adhE*) are deleted to block the production of alcohols.

Insertion of the operon in the *Clostridium* chromosome and deletion of the previously cited genes can be done using the method recently described in patent application PCT/EP2006/066997.

Advantageously, this *Clostridium* strain is selected among the group consisting of *Clostridium thermocellum, Clostridium saccharolyticum (now Thermoanaerobacter saccharolyticum), Clostridium thermosulfurogenes (now Thermoanaerobacter thermosulfurigenes) or Clostridium thermohydrosulfuricum (now Thermoanaerobacter ethanolicus*).

In a specific embodiment of the invention, the *Clostridium* strain has a decreased flux of hydrogen production and consequently presents a redirection of the flux of reducing equivalent toward 1,3-propanediol production. This result may be achieved by various means, and in particular by attenuating the gene encoding the hydrogenase (*hydA*), an enzyme that provides a sink for reducing equivalent in the form of hydrogen production. Attenuation of *hydA* can be done by replacing the natural promoter by a low strength promoter or by using an element destabilizing the corresponding messenger RNA or the protein. If needed, complete attenuation of the gene can also be achieved by partial or complete deletion of the corresponding DNA sequence.

In another embodiment of the invention, the used *Clostridium* strain presents an increased flux of 1,3-propanediol production; this result is achieved by introducing extra copies of the 1,3-propanediol operon from *C*. *butyricum,* (coding for enzymes involved in the B12 independent 1,3-propanediol pathway) either over-expressed by a plasmid or integrated into the chromosome of the recombinant *Clostridium.* For example the pSPD5 plasmid can be used for an over-expression of the 1,3-propanediol operon.

In another aspect of the invention, the *Clostridium* strain is modified to be able to convert acetate to acetone. This modification can be obtained by introducing into the microorganism an artificial "acetone operon" containing the *thl, ctfAB* and *adc* genes coding respectively for the thiolase, the CoA-transferase and the aceto-acetate decarboxylase, these three enzymes being involved in acetone formation in *C*. *acetobutylicum* and *C*. *beijerinckii.* This artificial operon can be either carried by a plasmid or can be integrated into the chromosome of the transformed *Clostridium.*

In another embodiment, the invention provides a method for the fermentative preparation of 1 ,3-propanediol at high yield, comprising:
(a) contacting a *Clostridium* strain with glycerol for a fermentation process whereby 1,3-propanediol is produced,
(b) isolating 1,3-propanediol and optionally a single oxidized product of the glycerol metabolism (mainly acetate or acetone) by distillation.

The fermentation is generally conducted in fermentors with an inorganic culture medium of known defined composition adapted to the bacteria used, containing at least glycerol, and if necessary a co-substrate necessary for the production of the metabolite.

This process can be realized in a batch process as well as in a continuous process. The man skilled in the art knows how to manage each of these experimental conditions, and to define the culture conditions for the microorganisms according to the invention. In particular the clostridia are fermented at a temperature between 20°C and 60°C, preferentially between 25°C and 40°C for mesophilic clostridia and between 45 and 60°C for thermophilic *Clostridia.*

The invention is also related to the microorganism as described previously. Preferably, this microorganism is selected among the group consisting of *C*. *butyricum, C. pasteurianum, C. acetobutylicum, C. beijerinckii, C. saccharoperbutylacetonicum C. saccharobutylicum, C. butyricum, C. cellulolyticum, Clostridium thermocellum, Clostridium saccharolyticum (now Thermoanaerobacter saccharolyticum), Clostridium thennosulfurogenes (now Thermoanaerobacter thermosulfurigenes) or Clostridium thermohydrosulfuricum (now Thermoanaerobacter ethanolicus).*

### EXAMPLE 1

### Construction of a recombinant Clostridium acetobutylicum producing 1,3-propanediol and unable to produce butyrate and butanol: C. acetobutylicumΔpSOL1 Δcac1515 Δupp Δbuk::PDO

To obtain a strain that can be genetically manipulated and is unable to produce butanol and acetone, we first cure the pSOL1 megaplasmid from the *C*. *acetobutylicum* Δcac1515 Δupp strain (described in patent application PCT/EP2006/066997) by i) running 20 sub-cultures in glucose MS medium and ii) by selection on agar plates (containing starch (2%) and glucose (0.2%) as described by Sabathe et al (2003)) of clones producing small hallo of starch hydrolysis to identify a *C*. *acetobutylicum* ΔpSOL1 Δcac1515 Δupp strain. To delete the *buk* gene and introduce the 1,3-propanediol operon from *C*. *butyricum,* the homologous recombination strategy described by Croux & Soucaille (2006) in patent application PCT/EP2006/066997 is used. A *buk* deletion cassette integrating the 1,3-propanediol operon from *C*. *butyricum* in the pCons::upp vector was constructed as follows.
Two DNA fragments surrounding *buk* were PCR amplified with the Pwo polymerase with total DNA from *C*. *acetobutylicum* as template and two specific couples of olignonucleotides. With the couples of primers BUK 1-BUK 21 and BUK 31-BUK 4, two DNA fragments were respectively obtained. Both primers BUK 1 and BUK 4 introduce a BamHI site while primers BUK 21 and BUK 31 have a complementary region which introduces pvuII and NruI sites. DNA fragments BUK 1-BUK 21 and BUK 31-BUK 4 were joined in a PCR fusion experiment with primers BUK 1 and BUK 4 and the resulting fragment was cloned in pCR4-TOPO-Blunt to yield pTOPO :buk. At the unique nruI site of pTOPO :buk, an antibiotic resistance MLS gene with FRT sequences on both sides was introduced from the 1372 bp StuI fragment of pUC18-FRT-MLS2. The BUK deletion cassette obtained after BamHI digestion of the resulting plasmid was cloned into pCons::upp at the BamHI site to yield the pREPΔBUK::upp plasmid. At the unique pvuII site of pREPΔBUK::upp, the 1,3-propanediol operon was introduced as a 4854bp blunt end Klenow treated SalI fragment of pSPD5 plasmid.
The pREPΔBUK::PDO::upp plasmid was used to transform by electroporation C. *acetobutylicum ΔpSOL1Δcac15Δupp* strain. After selection on Petri plate for clones resistant to erythromycin (40 µg/ml), one colony was cultured for 24 hours in Glycerol liquid synthetic medium with erythromycin at 40 µg/ml and 100 µl of undiluted culture was plated on RCGA (Reinforced *Clostridium* medium where starch and glucose are replaced by glycerol as a carbon source) with erythromycin at 40 µg/ml and 5-FU at 400 µM. Colonies resistant to both erythromycin and 5-FU were replica plated on both RCA with erythromycin at 40 µg/ml and RCA with thiamphenicol at 50 µg/ml to select clones where 5-FU resistance is also associated with thiamphenicol sensitivity. The genotype of clones resistant to erythromycin and sensitive to thiamphenicol was checked by PCR analysis (with primers BUK 0 and BUK 5 located outside of the *buk* deletion cassette).
The *ΔpSOL1Δcac15ΔuppΔbuk::PDO::mls^{R}* strain which have lost pREPΔbuk::upp was isolated.
The *ΔpSOL1Δcac15ΔuppΔbuk::PDO::mls^{R}* strain was transformed with pCLF1.1 vector expressing the *Flp1* gene encoding the Flp recombinase from *S*. *cerevisiae.* After transformation and selection for resistance to thiamphenicol (50 µg/ml) on Petri plate, one colony was cultured on synthetic liquid medium with thiamphenicol at 50 µg/ml and appropriate dilutions were plated on RCA with thiamphenicol at 50 µg/ml. Thiamphenicol resistant clones were replica plated on both RCA with erythromycin at 40 µg/ml and RCA with thiamphenicol at 50 µg/ml. The genotype of clones with erythromycin sensitivity and thiamphenicol resistance was checked by PCR analysis with primers BUK 0 and BUK 5. Two successive 24 hours cultures of the *Δcac15ΔuppΔbuk* strain with erythromycin sensitivity and thiamphenicol resistance were carried out in order to lose pCLF1.1. The *ΔpSOL1Δcac15ΔuppΔbuk::PDO* strain which has lost pCLF1.1 was isolated according to its sensitivity to both erythromycin and thiamphenicol.

**Table 1**

| Name | Primer sequences |
|---|---|
| Buk 1 | aaaaggatcc**tagtaaaagggagtgtacgaccagtg** |
| Buk 21 | ggggcagctggtcgcgaaaaaaggggg**gattattagtaatctatacatgttaacattcctccac** |
| Buk 31 | cccccttttttcgcgacccagctgcccc**acttcttgcacttgcagaaggtggac** |
| Buk 4 | aaaaggatcc**tctaaattctgcaatatatgccccccc** |
| Buk 0 | ataacaggatatatgctctctgacgcgg |
| Buk 5 | gatcatcactcattttaaacatggggcc |

### EXAMPLE 2

### Construction of strains unable to produce butyrate, acetone and lactate: C. acetobuylicum ΔpSOL1 Δcac1515 Δupp Δbuk::PDO Δldh

To delete the *ldh* gene, the homologous recombination strategy described by Croux & Soucaille (2006) in patent application PCT/EP2006/066997 is used. This strategy allows the insertion of an erythromycin resistance cassette, while deleting most of the genes concerned. The *ldh* deletion cassette in pCons::upp was constructed as follows.
Two DNA fragments surrounding *ldh* (*CAC267*) were PCR amplified with the Pwo polymerase with total DNA from *C*. *acetobutylicum* as template and two specific couples of olignonucleotides. With the couples of primers LDH 1-LDH 2 and LDH 3-LDH 4, 1135 bp and 1177 bp DNA fragments were respectively obtained. Both primers LDH 1 and LDH 4 introduce a BamHI site while primers LDH 2 and LDH 3 have a complementary region which introduces a StuI site. DNA fragments LDH 1-LDH 2 and LDH 3-LDH 4 were joined in a PCR fusion experiment with primers LDH 1 and LDH 4 and the resulting fragment was cloned in pCR4-TOPO-Blunt to yield pTOPO :LDH. At the unique StuI site of pTOPO :LDH, an antibiotic resistance MLS gene with FRT sequences on both sides was introduced from the 1372 bp StuI fragment of pUC18-FRT-MLS2. The UPP deletion cassette obtained after BamHI digestion of the resulting plasmid was cloned into pCons::upp at the BamHI site to yield the pREPΔLDH::upp plasmid.
The pREPΔLDH::upp plasmid was used to transform by electroporation *C*. *acetobutylicum ΔpSOL1*Δ*cac15*Δ*upp*Δ*bu::PDO* strain. After selection on Petri plate (on RCGA) for clones resistant to erythromycin (40 µg/ml), one colony was cultured for 24 hours in liquid glycerol synthetic medium with erythromycin at 40 µg/ml and 100 µl of undiluted culture was plated on RCGA with erythromycin at 40 µg/ml and 5-FU at 400 µM. Colonies resistant to both erythromycin and 5-FU were replica plated on both RGCA with erythromycin at 40 µg/ml and RGCA with thiamphenicol at 50 µg/ml to select clones where 5-FU resistance is also associated with thiamphenicol sensitivity. The genotype of clones resistant to erythromycin and sensitive to thiamphenicol was checked by PCR analysis (with primers LDH 0 and LDH 5 located outside of the *ldh* deletion cassette). The *ΔΔpSOL1*Δ*cac15*Δ*upp*Δ*buk::PDO*Δ*ldh::mls^{R}* strain which have lost pREPΔLDH::upp was isolated.
The Δ*pSOL1*Δ*cac15*Δ*upp*Δ*buk::PDO*Δ*ldh::mls^{R}* strain was transformed with pCLF1.1 vector expressing the *Flp1* gene encoding the Flp recombinase from *S*. *cerevisiae.* After transformation and selection for resistance to thiamphenicol (50 µg/ml) on Petri plate, one colony was cultured on synthetic liquid medium with thiamphenicol at 50 µg/ml and appropriate dilutions were plated on RCA with thiamphenicol at 50 µg/ml. Thiamphenicol resistant clones were replica plated on both RCA with erythromycin at 40 µg/ml and RCA with thiamphenicol at 50 µg/ml. The genotype of clones with erythromycin sensitivity and thiamphenicol resistance was checked by PCR analysis with primers LDH 0 and LDH 5. Two successive 24 hours cultures of the Δ*pSOL1*Δ*cac15*Δ*uppΔbuk::PDO*Δ*ldh* strain with erythromycin sensitivity and thiamphenicol resistance were carried out in order to lose pCLF1.1. The Δ*pSOL1*Δ*cac15*Δ*upp*Δ*buk::PDO*Δ*ldh* strain which has lost pCLF1.1 was isolated according to its sensitivity to both erythromycin and thiamphenicol.

**Table 2**

| Name | Primer sequences |
|---|---|
| Ldh 1 | AAAAGGATCCGCTTTAAAATTTGGAAAGAGGAAGTTGTG |
| Ldh 2 | GGGGAGGCCTAAAAAGGGGGTTAGAAATCTTTAAAAATTTCTCTATAGAGCCCATC |
| Ldh 3 | CCCCCTTTTTAGGCCTCCCCGGTAAAAGACCTAAACTCCAAGGGTGGAGGCTAGGTC |
| Ldh 4 | AAAAGGATCCCCCATTGTGGAGAATATTCCAAAGAAGAAAATAATTGC |
| Ldh 0 | CAGAAGGCAAGAATGTATTAAGCGGAAATGC |
| Ldh 5 | CTTCCCATTATAGCTCTTATTCACATTAAGC |

### EXAMPLE 3

### Construction of strains with lower hydrogen production: C. acetobutylicum ΔpSOL1 Δcac1515 Δupp Δbuk::PDO Δldh ΔhydA

To delete the *hydA* gene, the homologous recombination strategy described by Croux & Soucaille (2006) in patent application PCT/EP2006/066997 is used. This strategy allows the insertion of an erythromycin resistance cassette, while deleting most of the genes concerned. The *hydA* deletion cassette in pCons::upp was constructed as follows.
Two DNA fragments surrounding *hydA* (*CAC028*) were PCR amplified with the Pwo polymerase with total DNA from *C*. *acetobutylicum* as template and two specific couples of olignonucleotides. With the couples of primers HYD 1-HYD 2 and HYD 3-HYD 4, 1269 bp and 1317 bp DNA fragments were respectively obtained. Both primers HYD 1 and HYD 4 introduce a BamHI site while primers HYD 2 and HYD 3 have a complementary region which introduces a StuI site. DNA fragments HYD 1-HYD 2 and HYD 3-HYD 4 were joined in a PCR fusion experiment with primers HYD 1 and HYD 4 and the resulting fragment was cloned in pCR4-TOPO-Blunt to yield pTOPO :HYD. At the unique StuI site of pTOPO :HYD, an antibiotic resistance MLS gene with FRT sequences on both sides was introduced from the 1372 bp StuI fragment of pUC18-FRT-MLS2. The UPP deletion cassette obtained after BamHI digestion of the resulting plasmid was cloned into pCons::upp at the BamHI site to yield the pREPΔHYD::upp plasmid.
The pREPΔHYD::upp plasmid was used to transform by electroporation *C*. *acetobutylicum ΔpSOL1Δcac15ΔuppΔbuk::PDOΔldh* strain. After selection on Petri plate (RCGA) for clones resistant to erythromycin (40 µg/ml), one colony was cultured for 24 hours in glycerol liquid synthetic medium with erythromycin at 40 µg/ml and 100 µl of undiluted culture was plated on RCA with erythromycin at 40 µg/ml and 5-FU at 400 µM. Colonies resistant to both erythromycin and 5-FU were replica plated on both RCGA with erythromycin at 40 µg/ml and RCA with thiamphenicol at 50 µg/ml to select clones where 5-FU resistance is also associated with thiamphenicol sensitivity. The genotype of clones resistant to erythromycin and sensitive to thiamphenicol was checked by PCR analysis (with primers HYD 0 and HYD 5 located outside of the *hydA* deletion cassette). The Δ*pSOL1*Δ*cac15*Δ*upp*Δ*buk::PDO*Δ*ldh*Δ*hydA::mls^{R}* strain which have lost pREPΔHYD::upp was isolated.
The Δ*pSOL1*Δ*cac15*Δ*upp*Δ*buk::PDO*Δ*ldh*Δ*hydA::mls^{R}* strain was transformed with pCLF1.1 vector expressing the *Flp1* gene encoding the Flp recombinase from S. *cerevisiae.* After transformation and selection for resistance to thiamphenicol (50 µg/ml) on Petri plate, one colony was cultured on synthetic liquid medium with thiamphenicol at 50 µg/ml and appropriate dilutions were plated on RCA with thiamphenicol at 50 µg/ml. Thiamphenicol resistant clones were replica plated on both RCA with erythromycin at 40 µg/ml and RCA with thiamphenicol at 50 µg/ml. The genotype of clones with erythromycin sensitivity and thiamphenicol resistance was checked by PCR analysis with primers HYD 0 and HYD 5. Two successive 24 hours cultures of the Δ*pSOL1*Δ*cac15*Δ*pp*Δ*buk::PDO*Δ*ldh*Δ*hydA* strain with erythromycin sensitivity and thiamphenicol resistance were carried out in order to lose pCLF1.1. The Δ*pSOL1*Δ*cac15*Δ*upp*Δ*buk::PDO*Δ*ldh*Δ*hydA* strain which has lost pCLF1.1 was isolated according to its sensitivity to both erythromycin and thiamphenicol.

**Table 3**

| Name | Primer sequences |
|---|---|
| Hyd 1 | AAAA*GGATCCGCC*TCTTCTGTATTATGCAAGGAAAGCAGCTGC |
| Hyd 2 | GGGGAGGCCTAAAAGGGGGTATATAAAATAAATGTGCCTTAACATCTAA GTTGAGGCC |
| Hyd 3 | CCCCCTTTTTAGGCCTCCCCGTTATCCTCCCAAAATGTAAAATATAATTAA AATATATTAATAAACTTCGATTAATAAACTTCG |
| Hyd 4 | AAAA*GGATCC*CCTTTTAGCGTATAAAGTTTTATATAGCTATTG |
| Hyd 0 | CATGTTCTATTGTTACTATGGAAGAGGTAGTAG |
| Hyd 5 | GCAGTTATTATAAATGCTGCTACTAGAGC |

### EXAMPLE 4

### Construction of strains with increase flux in the 1,3-propanediol pathway: C. acetobutylicum ΔpSOL1 Δcac1515 Δupp Δbuk::PDO Δldh pSPD5.

To construct a strain that converts glycerol to 1,3-propanediol and acetate at higher flux we introduce the pSPD5 plasmid (described in patent application WO01/04324) that expressed as an operon the B12 independent 1,3-propanediol pathway from *C*. *butyricum.* The pSPD5 plasmid was used to transform by electroporation of *C*. *acetobutylicum ΔpSOL1Δcac15ΔuppΔbuk::PDOΔldh* strain. After selection on Petri plate (RCGA) for clones resistant to erythromycin (40 µg/ml), one colony was cultured for 24 hours in glycerol liquid synthetic medium with erythromycin at 40 µg/ml and use to extract the pSPD5 plasmid that was characterized by its restriction profile.

### EXAMPLE 5

### Construction of strains producing 1,3-propanediol and acetone: C. acetobutylicum ΔpSOL1 Δcac1515 Δupp Δbuk::PDO Δldh pSOS95 thl.

To construct a strain that converts acetate to acetone the pSOS 95 thl plasmid expressing a synthetic acetone operon was constructed. For this purpose the thl gene encoding thiolase (from *C*. *acetobutylicum*) was introduced at the BamHI site of the pSOS95 vector (genbank accession n° AY 187686) already expressing as a synthetic operon the ctfAB and adc genes. The thl gene was PCR amplified with the Pwo polymerase with total DNA from C. *acetobutylicum* as template and two specific couples of olignonucleotides. With the couple of primers THL 1-THL2 a 1.2 kbp DNA fragment was obtained and digested with BamHI and BglII, two restriction sites that were respectively introduced by primers THL 1 and THL2. After ligation to the BamhI digested pSOS95, the pSOS95-thl plasmid was obtained.
This pSOS95-thl plasmid was used to transform by electroporation *C*. *acetobutylicum* Δ*pSOL1*Δ*cac15*Δ*upp*Δ*buk::PDO*Δ*ldh* strain. After selection on Petri plate (RCGA) for clones resistant to erythromycin (40 µg/ml), one colony was cultured for 24 hours in glycerol liquid synthetic medium with erythromycin at 40 µg/ml and use to extract the pSOS95-thl plasmid that was characterized by its restriction profile.

**Table 4**

| Name | Primer sequences |
|---|---|
| THL 1 | cgcggatcctttatctgttaccccgtatcaaaatttagg |
| THL 2 | gaAGATCTTCTAGCACTTTTCTAGCAATATTGC |

### EXAMPLE 6

### Batch fermentation of 1,3-propanediol producing strains.

Strains were initially analyzed in anaerobic flask cultures in the synthetic medium described by Soni et al (Soni et al, 1986, Appl. Microbiol.Biotechnol. 32:120-128) supplemented with 2.5 g/l of ammonium acetate and with replacement of glucose by glycerol. An overnight culture at 35°C was used to inoculate a 30 ml culture to an OD600 of 0.05. After incubation of the culture for 3 days at 35°C, glycerol, organic acids and 1,3-propanediol were analyzed by HPLC using a Biorad HPX 97H column for the separation and a refractometer for the detection.

Strains with the correct phenotype were subsequently tested under production conditions in 300 ml fermentors (DASGIP) using an anaerobic batch protocol.

For this purpose the fermentor was filled with 250 ml of synthetic medium, sparged with nitrogen for 30 min and inoculated with 25 ml of preculture to an optical density (OD600nm) between 0.05 and 0.1.

The temperature of the culture was maintained constant at 35 °C and the pH was permanently adjusted at 6.5 using an NH₄OH solution. The agitation rate was maintained at 300 rpm during the fermentation.

### EXAMPLE 7

### Continuous fermentation of 1,3-propanediol and acetate producing strains.

The best 1,3-propanediol and acetate producing strain was analyzed in chemostat cultures in the synthetic medium described by Soni et al (Soni et al, 1987, Appl. Microbiol.Biotechnol.) except that glucose was replaced by glycerol. An overnight culture at 35°C was used to inoculate a 300 ml fermentors (DASGIP) using an anaerobic chemostat protocol.

For this purpose the fermentor was filled with 250 ml of synthetic medium, sparged with nitrogen for 30 min and inoculated with 25 ml of preculture to an optical density (OD600nm) between 0.05 and 0.1. After 12 hours of batch culture at 35 °C, pH 6.5 (regulated using an NH₄OH solution) and an agitation rate of 300 rpm, the fermentor was continuously fed with oxygen free synthetic medium at a dilution rate of 0.05 h-1 while the volume was kept constant by sequential removal of fermentated medium. Stability of the culture was followed by products analysis using the HPLC protocol previously described.

### REFERENCES

Gonzalez-Pajuelo M, Meynial-Salles I, Mendes F, Andrade JC, Vasconcelos I, Soucaille P. Metabolic engineering of Clostridium acetobutylicum for the industrial production of 1,3-propanediol from glycerol.
Metab Eng. 2005,;7:329-36..

### Green EM, Boynton ZL, Harris LM, Rudolph FB, Papoutsakis ET, Bennett GN.

Genetic manipulation of acid formation pathways by gene inactivation in Clostridium acetobutylicum ATCC 824.
Microbiology. 1996,142 :2079-86.

Soni B. K., Soucaille P. Goma G. Continuous acetone butanol fermentation : influence of vitamins on the metabolic activity of Clostridium acetobutylicum. Appl. Microbiol. Biotechnol. 1987. 27 : 1-5.

## Claims

1. A method for the anaerobic production of 1, 3 propanediol, by culturing a *Clostridium* strain in an appropriate culture medium comprising glycerol as a source of carbon, and recovering 1,3-propanediol,
wherein in said *Clostridium* strain, at least one gene coding for an enzyme involved in butyrate production is deleted, and at least one heterologous gene coding for an enzyme involved in the B12 independent 1,3-propanediol pathway was introduced.

2. The method of claim 1, wherein the gene involved in butyrate formation is selected among :
• *ptb* encoding phospho-transbutyrylase
• *buk* encoding butyrate kinase.

3. A method according to claim 1 or 2, wherein the enzyme involved in the B12 independent 1,3-propanediol pathway is encoded by an operon from *Clostridium butyricum.*

4. A method according to anyone of claims 1 to 3, wherein in said Clostridium strain, the *ldh* gene coding for lactate dehydrogenase is deleted.

5. A method according to claims 1 to 4, wherein in said Clostridium strain, the *hydA* gene is attenuated.

6. A method according to claims 1 to 5, wherein in said Clostridium strain, the *adhE* gene coding for aldehyde-alcohol dehydrogenase is deleted.

7. The method according to anyone of claims 1 to 6, wherein the Clostridium strain is **selected** among the group consisting of *C*. *pasteurianum, C. acetobutylicum, C. beijerinckii, C. saccharoperbutylacetonicum C. saccharobutylicum, C. butyricum* or *C. cellulolyticum.*

8. The method of claim 7, wherein the Clostridium strain is selected among the group consisting of *C*. *butyricum* and *C*. *pasteurianum.*

9. A method as claimed in any one of claims 1 to 8, wherein the culture is continuous.

10. A method as claimed in any one of claims 1 to 8, wherein the culture is made in batchs.

11. A microorganism as defined in any one of claims 1 to 8.


SEQUENCE LISTING

<110>  METABOLIC EXPLORER

<120>  process for the biological production of 1,3-propanediol from
       glycerol with high yield

<130>  69430 D24893 LUV

<150>  EP 06819204.6
<151>  2006 10 31

<150>  PCT/EP2006/067987
<151>  2006-10-31

<160>  20

<170>  PatentIn version 3.3

<210>  1
<211>  36
<212>  DNA
<213>  Artificial

<220>
<223>  Buk 1

<400>  1
aaaaggatcc tagtaaaagg gagtgtacga ccagtg                                36


<210>  2
<211>  64
<212>  DNA
<213>  Artificial

<220>
<223>  Buk 21

<400>  2
ggggcagctg gtcgcgaaaa aaggggggat tattagtaat ctatacatgt taacattcct      60

ccac                                                                   64


<210>  3
<211>  53
<212>  DNA
<213>  Artificial

<220>
<223>  Buk 31

<400>  3
   cccccttttt tcgcgaccag ctgccccact tcttgcactt gcagaaggtg gac            53


<210>  4
<211>  37
<212>  DNA
<213>  Artificial

<220>
<223>  Buk 4

<400>  4
aaaaggatcc tctaaattct gcaatatatg ccccccc                                37

<210>  5
<211>  28
<212>  DNA
<213>  Artificial

<220>
<223>  Buk 0

<400>  5
ataacaggat atatgctctc tgacgcgg                                          28

<210>  6
<211>  28
<212>  DNA
<213>  Artificial

<220>
<223>  Buk 5

<400>  6
gatcatcact cattttaaac atggggcc                                          28

<210>  7
<211>  39
<212>  DNA
<213>  Artificial

<220>
<223>  Ldh 1

<400>  7
aaaaggatcc gctttaaaat ttggaaagag gaagttgtg                              39

<210>  8
<211>  56
<212>  DNA
<213>  Artificial

<220>
<223>  Ldh 2

<400>  8
ggggaggcct aaaaaggggg ttagaaatct ttaaaaattt ctctatagag cccatc          56

<210>  9
<211>  57
<212>  DNA
<213>  Artificial

<220>
<223>  Ldh 3

<400>  9
cccccttttt aggcctcccc ggtaaaagac ctaaactcca agggtggagg ctaggtc        57

<210>  10

<211>  48
<212>  DNA
<213>  Artificial

<220>
<223>  Ldh 4

<400>  10
aaaaggatcc cccattgtgg agaatattcc aaagaagaaa ataattgc                    48


<210>  11
<211>  31
<212>  DNA
<213>  Artificial

<220>
<223>  Ldh 0

<400>  11
cagaaggcaa gaatgtatta agcggaaatg c                                      31


<210>  12
<211>  31
<212>  DNA
<213>  Artificial

<220>
<223>  Ldh 5

<400>  12
cttcccatta tagctcttat tcacattaag c                                      31


<210>  13
<211>  43
<212>  DNA
<213>  Artificial

<220>
<223>  Hyd 1

<400>  13
aaaaggatcc gcctcttctg tattatgcaa ggaaagcagc tgc                         43


<210>  14
<211>  59
<212>  DNA
<213>  Artificial

<220>
<223>  Hyd 2

<400>  14
ggggaggcct aaaaaggggg tatataaaat aaatgtgcct taacatctaa gttgaggcc        59


<210>  15
<211>  85
<212>  DNA
<213>  Artificial

<220>

<223>  Hyd 3

<400>  15
cccccttttt aggcctcccc gtttatcctc ccaaaatgta aaatataatt aaaatatatt      60

aataaacttc gattaataaa cttcg                                              85


<210>  16
<211>  43
<212>  DNA
<213>  Artificial

<220>
<223>  Hyd 4

<400>  16
aaaaggatcc ccttttagcg tataaagttt tatatagcta ttg                         43


<210>  17
<211>  33
<212>  DNA
<213>  Artificial

<220>
<223>  Hyd 0

<400>  17
catgttctat tgttactatg gaagaggtag tag                                     33


<210>  18
<211>  29
<212>  DNA
<213>  Artificial

<220>
<223>  Hyd 5

<400>  18
gcagttatta taaatgctgc tactagagc                                          29


<210>  19
<211>  39
<212>  DNA
<213>  Artificial

<220>
<223>  THL 1

<400>  19
cgcggatcct ttatctgtta ccccgtatca aaatttagg                               39


<210>  20
<211>  33
<212>  DNA
<213>  Artificial

<220>
<223>  THL 2

<400>  20

gaagatcttc tagcactttt ctagcaatat tgc 33